# EUROPEAN PATENT APPLICATION

(11) **EP 2 255 823 A1**
(43) Date of publication of application: **01.12.2010**
(21) Application number: 09710558.9
(22) Date of filing: 16.02.2009
(51) Int. Cl.: A61K 38/00, A23L 1/30, A61P 1/00, A61P 1/12, A61P 31/00, A61P 35/00, A61P 37/04, A61P 37/08

(54) **INTESTINAL IMMUNE SYSTEM STIMULATOR**

(30) Priority: 15.02.2008 JP 2008034820
(71) Applicant: Ajinomoto Co., Inc., Tokyo 104-8315 (JP)
(72) Inventor: KODERA, Tomohiro, Kawasaki-shi Kanagawa 210-8681 (JP); NIO, Noriki, Tokyo 104-8315 (JP); ONISHI, Norimasa, Tokyo 104-8315 (JP); MINE, Yoshinori, Shizuoka-shi Shizuoka 421-0101 (JP)
(74) Representative: Fischer, Heinrich
(86) International application number: PCT/JP2009/052462
(87) International publication number: WO 2009/102050

(57) **Abstract**

Disclosed is an intestinal immune system stimulator which can stimulate an intestinal immune system effectively and is safe. γ-Glutamylcysteine is used as an active ingredient of an intestinal immune system stimulator which can be used as a medicinal agent for the treatment or prevention of infectious diseases, diarrhea, polyps, tumors, enteritis or allergy. A food having an intestinal immune system-stimulating activity can be produced by adding γ-glutamylcysteine to a food.

## Description

### Technical Field

The present invention relates to an intestinal immune system stimulator, which can be used in the fields of pharmaceuticals, foods, and the like.

### Background Art

γ-Glutamylcysteine (γ-Glu-Cys, γ-EC) is a dipeptide which is a precursor of glutathione. Food materials containing a high level of cysteine can be obtained by heating or enzymatically treating a yeast extract containing γ-glutamylcysteine (Patent Document 1).

Meanwhile, it has been suggested that γ-glutamylcysteine and its derivatives or analogs have an action of activating a calcium sensing receptor (CaSR), and these CaSR agonists can be used as therapeutic agents for medical diseases (Patent Document 2).

Moreover, there has been disclosed an antirheumatic agent containing a substance having an action of decreasing the amount of reduced glutathione in macrophage cells to suppress production of IL-12, production of NO, and production of INF-γ, resulting in suppression of a delayed hypersensitivity reaction, and it has been reported that compounds such as γ-glutamylcysteine or γ-glutamylcysteine diethyl ester are substances capable of increasing the amount of reduced glutathione in macrophage cells (Patent Document 3).

Furthermore, there has been disclosed an immunomodulator containing a substance having an action of altering the amount of reduced glutathione in macrophage cells, and an example of the substances includes γ-glutamylcysteine diethyl ester (Patent Document 4) . In addition, there has been disclosed an antidiabetic agent containing a substance having an action of altering the amount of reduced glutathione in macrophage cells, and examples of the substance include γ-glutamylcysteine and γ-glutamylcysteine dimethyl ester (Patent Document 5).

Meanwhile, there has been known an agent for preventing infections containing a combination of cystine, which is a cysteine compound, or a derivative thereof with theanine (Patent Document 6).

However, it has not been known that γ-glutamylcysteine can remarkably stimulate the intestinal immune system. Furthermore, as mentioned above, it has been known that a yeast extract or the like containing γ-glutamylcysteine is used as a food material to manufacture a food containing cysteine, but a food containing, in a final form, a significant amount of γ-glutamylcysteine has been not known.
Patent Document 1: WO 00/30474 A1
Patent Document 2: WO 2007/55388 A2
Patent Document 3: JP 2000-309532 A
Patent Document 4: JP 11-246435 A
Patent Document 5: JP 2000-309543 A
Patent Document 6: WO 2003/068214 A1

### Disclosure of the Invention

An object of the present invention is to provide an intestinal immune system stimulator which can effectively and safely stimulate the intestinal immune system, and a food or a beverage containing the stimulator.

The inventors of the present invention have made intensive studies to solve the above-described problem. As a result, the inventors have found that γ-glutamylcysteine has an action of stimulating the intestinal immune system and thus have completed the present invention.
That is, the present invention is as follows.
(1) An intestinal immune system stimulator comprising γ-glutamylcysteine.
(2) The intestinal immune system stimulator, which is used as a medicament for the treatment or prevention of infection, diarrhea, polyp, tumor, enteritis, or allergy.
(3) A food or beverage comprising γ-glutamylcysteine.
(4) The food or beverage comprising γ-glutamylcysteine in an amount of 0.000001% or more by mass.
(5) The food or beverage according to the item (3) or (4), which has an effect of stimulating the intestinal immune system.
(6) A use of γ-glutamylcysteine for manufacturing an intestinal immune system stimulator.
(7) The use according to the item (6), wherein the intestinal immune system stimulator is a medicament for the treatment or prevention of diarrhea, polyp, tumor, enteritis, or allergy.
(8) Amethod for stimulating the intestinal immune system, comprising administering γ-glutamylcysteine to a subject in need of stimulation of the intestinal immune system.
(9) A method for treating or preventing infection, diarrhea, polyp, tumor, enteritis, or allergy, comprising administering γ-glutamylcysteine to a subject having infection, diarrhea, polyp, tumor, enteritis, or allergy.

### Brief Description of the Drawings

FIG. 1 is a graph illustrating the amounts of IgAproduced when stimulating mouse spleen cells with LPS. In this graph, EC represents γ-glutamylcysteine, and GSH represents glutathione (the same is true for the following figures).
FIG. 2 is a graph illustrating the amounts of IgG produced when stimulating mouse spleen cells with LPS.
FIG. 3 is a graph illustrating the amounts of IgA produced when stimulating mouse spleen cells with ConA.
FIG. 4 is a graph illustrating the amounts of IgG produced when stimulating mouse spleen cells with ConA.

### DESCRIPTION OF THE PREFERRED EMBODIMENTS

Hereinafter, the present invention is described in detail.
An intestinal immune system stimulator of the present invention contains γ-glutamylcysteine.
γ-glutamylcysteine can be prepared from, for example, a yeast containing γ-glutamylcysteine.

The yeast containing γ-glutamylcysteine includes:
a yeast which overexpresses γ-glutamylcysteine synthetase (Otake et al., Bioscience and Industry, 50(10), 989-994, 1992);
a yeast in which glutathione synthetase has been modified not to function by disrupting the glutathione synthetase gene (WO 00/30474 A1); a yeast which harbors a mutant glutathione synthetase having one or both mutations selected from a mutation replacing the threonine residue with an isoleucine residue at position 47 and a mutation replacing the glycine residue with an aspartic acid residue at position 387, and which produces γ-glutamylcysteine (US 2003159049, EP 1449913);
a yeast having a γ-glutamylcycteine-producing ability and harboring a mutant MET30 gene which encodes a protein having a mutation replacing the serine at position 569 with an amino acid other than serine (JP 2004-113155 A);
*Saccharomyces cerevisiae* in which glutathione synthetase encoded by the glutathione synthetase gene on the chromosome has a deletion of the C-terminus region from the arginine residue at position 370 (US 2003124684, EP 1201747); and
*Candida utilis* in which the gene encoding glutathione synthetase has been modified to decrease intracellular glutathione, synthetase activity (EP 1489173).

Furthermore, methods for obtaining a yeast having a γ-glutamylcycteine-producing ability includes:
a method comprising selecting yeast strains which are resistant to a certain concentration range of MNNG from a yeast population, and selecting, from the selected strains, a strain having attenuated glutathione synthetase activity and having the γ-glutamylcysteine-producing ability (US 2004214308, EP 1452585); and
a method comprising a step of culturing a yeast which is auxotrophic for pantothenic acid in a medium containing a sufficient amount of pantothenic acid to proliferate the yeast and a step of culturing the yeast in a medium having a limited amount of pantothenic acid to increase the content of γ-glutamylcysteine in the yeast cells (JP 2004-201677 A).

If the yeast which produces γ-glutamylcysteine as described above is cultured under suitable conditions, a yeast containing γ-glutamylcysteine or a culture containing the same can be obtained.
The medium to be used in the culture of the yeast is not particularly limited as long as the yeast can grow well and efficiently produce γ-glutamylcysteine, and generally, a medium typically used in industry can be used. It should be noted that, if necessary, necessary nutrients are added to the medium depending on the character of the yeast to be used.

For example, in the case of *Saccharomyces cerevisiae,* preferably, a culture broth obtained by culturing the yeast to the logarithmic growth phase or stationary phase is inoculated into a nutrient medium in an amount of 2%, and the yeast is cultured at 30°C for 10 to 30 hours.

The yeast culture obtained as above or a fraction thereof contains γ-glutamylcysteine. γ-Glutamylcysteine to be used in the present invention can be a purified product, or can be a yeast culture, yeast cells, a yeast cell homogenate, a yeast cell extract (yeast extract), or the like as long as γ-glutamylcysteine is contained. Moreover, a fraction containing γ-glutamylcysteine can be obtained from the yeast cell homogenate or yeast extract.

γ-Glutamylcysteine can be in the form of a salt. The salt can be any salt as long as it is pharmacologically acceptable, and examples include an ammonium salt; a salt with an alkali metal such as sodium or potassium; a salt with an alkaline-earth metal such as calcium or magnesium; an aluminum salt; a zinc salt; a salt with an organic amine such as triethylamine, ethanolamine, morpholine, pyrrolidine, piperidine, piperazine, or dicyclohexylamine; and a salt with a basic amino acid such as arginine or lysine.

The intestinal immune system stimulator of the present invention contains γ-glutamylcysteine as an active ingredient. Examples of the embodiments of the intestinal immune system stimulator of the present invention include pharmaceuticals, quasi drugs, and foods or beverages.

The intestines are known to be the largest immune organs, and overall immunity can be improved by stimulating the intestinal immune system. Therefore, the intestinal immune system stimulator of the present invention is effective for the treatment or prevention of diseases which are prevented or treated effectively by stimulating the intestinal immune system, for example, a variety of infections, diarrhea, polyps, tumors, enteritis, or allergies.

The infections include viral infections and bacterial infections. The viral infections are not particularly limited, and examples of the viral infections include gastrointestinal virus infections (infections caused by gastrointestinal viruses such as enterovirus and cytomegalovirus), respiratory virus infections (infections caused by respiratory viruses such as influenza virus, rhinovirus, coronavirus, parainfluenza virus, RS virus, adenovirus, and reovirus), herpes zoster caused by herpesvirus, diarrhea caused by rotavirus, viral hepatitis, and AIDS. The present invention is particularly effective for the gastrointestinal virus infections.

Furthermore, the bacterial infections are not particularly limited, and examples include infections caused by *Bacillus cereus, Vibrio parahaemolyticus,* enterohemorrhagic *Escherichia coli, Staphylococcus aureus,* MRSA, *Salmonella, Clostridium botulinum,* and *Candida.*

In the present invention, stimulation of the intestinal immune system means activation of the immune system which the intestines have independently. Specifically, for example, the stimulation means promotion of IgA secretion in the intestines, in particular, in the small intestine.

Depression of the immune system of the intestines causes diseases, and examples of symptoms/diseases which are consistently related to the immunity of the intestines include infections, allergic diseases, polyps, tumors, and enteritis (Lecture on intestinal immune system,
http://www.ioudou.co.jp/col/archives/2004/11/post_7.html).
Meanwhile, as shown in the examples below, γ-glutamylcysteine has an action of promoting IgA secretion in the intestines, in particular, in the small intestine. Secretory IgA binds specifically to a microorganism such as an invasive bacterium or virus to inhibit adhesion of the microorganism to the epidermal cells. Moreover, secretory IgA is known to have a neutralization activity on toxins produced by bacteria such as *Vibrio cholerae,* and to bind to allergens contained in foods to inhibit absorption of the dietary antigen into the body (Hisako YASUI, Intestinal immune system-regulating action of bifidobacterium,
http://www.healthist.jp/special/150_03/03_03.html).

Moreover, secretory IgA is known to be involved in the induction of "oral immune tolerance", which is an immunosuppressive mechanism, for a protein absorbed from the intestines (Satoshi HACHIMURA, Intestinal immune system as contact between food and immune system: Unique cell responsiveness,
http://jsbba.bt.a.u-tokyo.ac.jp/03reikai3/hachimura.pdf), and allergy can be suppressed by induction of oral immune tolerance.

The above-described facts suggest that stimulation of the intestinal immune system, in particular, the promotion of IgA secretion in the intestines is effective for the treatment or prevention of the above-described diseases.

Methods for applying the intestinal immune system stimulator of the present invention are not particularly limited and oral administration, invasive administration using an injection or the like, suppository administration, or transdermal administration can be adopted. The active ingredient can be mixed with a solid or liquid non-toxic pharmaceutical carrier suitable for an administration method by oral route, injection, or the like to administer the ingredient in the form of a conventional pharmaceutical formulation. Examples of the formulation include: a form of a solid drug such as a tablet, a granule, a powder, or a capsule; a form of a liquid drug such as a solution, a suspension, or an emulsion; and a form of a freeze-dried drug. The formulation can be prepared by a conventional method for formulation.

Examples of the above-described non-toxic pharmaceutical carrier include glucose, lactose, sucrose, starch, mannitol, dextrin, fatty acid glyceride, polyethylene glycol, hydroxyethyl starch, ethylene glycol, polyoxyethylene sorbitan fatty acid ester, gelatin, albumin, amino acids, water, and physiological saline. Furthermore, if necessary, a commonly used additive such as a stabilizer, a humectant, an emulsifier, a binder, or a tonicity agent can also be appropriately added.

The intestinal immune system stimulator of the present invention can contain not only γ-glutamylcysteine but also one kind or two or more kinds of other medicaments effective for treatment of the target disease, such as a substance having an action of stimulating the intestinal immune system or an action of treating or preventing infections. Examples of such medicaments include cystine or a derivative thereof, the anine, and lactic acid bacteria.

The dosage or intake of the intestinal immune system stimulator of the present invention can be an amount effective for the treatment or prevention and is appropriately adjusted depending on the patient's age, sex, weight, symptom, etc. For example, in the case of oral administration, the amount of γ-glutamylcysteine per dose is preferably 0.0005 g to 1 g per kg body weight or more preferably 0.001 g to 0.2 g per kg body weight. The number of administrations is not particularly limited, and administration can be performed once to several times a day.

The content of γ-glutamylcysteine in the intestinal immune system stimulator of the present invention is not particularly limited as long as the content is suitable for the above-described dosage. The content is preferably 0.000001% by mass to 70% by mass, more preferably 0.00001% by mass to 50% by mass, or particularly preferably 0.0001% by mass to 40% by mass per dry weight.

The food or beverage of the present invention contains γ-glutamylcysteine. The kind of the food or beverage of the present invention is not particularly limited, and examples of the food or beverage include seasonings, a fermented food, an alcoholic beverage, soup, sauce, mayonnaise, dressing, curry roux, juice, a nutritional beverage, rice gruel, bread, confectionery, a retort pouch food, a frozen food, a supplement, a milk product, and a cosmetic food.

The food or beverage of the present invention can be manufactured by the same method using the same materials as for manufacturing a general food or beverage except that the food or beverage contains γ-glutamylcysteine. The materials are not particularly limited, and examples of the materials include: rice, barley, and corn starch for an alcohol beverage; flour, sugar, salt, butter, and fermentation yeast for bread; and soybean and wheat for a fermented food.

The food or beverage of the present invention contains γ-glutamylcysteine in an amount of 0.000001% or more by mass, preferably 0.01% or more by mass, more preferably 0.05% or more by mass, or particularly preferably 0.1% or more by mass. The upper limit of the content of γ-glutamylcysteine is not particularly limited but is preferably 50% or less by mass, more preferably 10% or less by mass, or particularly preferably 5% or less by mass.

The food or beverage of the present invention has an effect of stimulating the intestinal immune system, and for example, the container or package thereof can state that the food or beverage has the stimulatory effect or that the food or beverage has a therapeutic or preventive effect for the above-described diseases.

### Examples

Hereinafter, the present invention is described more specifically by way of examples. However, the present invention is not limited to the following examples. It should be noted that the γ-glutamylcysteine used in the examples was γ-glutamylcysteine obtained by custom synthesis and purification by PEPTIDE INSTITUTE, INC.

### [Example 1]

Six- to eight-week-old BALB mice (female) were purchased and fed for 1 week for habituation. A commercially-available solid feed was used as a feed, and tap water was used as water. To evaluate the influence of γ-glutamylcysteine on the antibody-producing activity, a culture system using mouse spleen cells was employed. The spleen cells were collected from the BALB-c mice (n=3). The collected cells were washed and then suspended in an RPMI 1640 medium (10% FCS, 50 U/ml penicillin, 50 µg/ml streptomycin). To cells adjustedto 1×10⁶ cells per 48-well culture plate, γ-glutamylcysteine or glutathione (purchased from Sigma) (each was dissolved in a phosphate buffer), or a phosphate buffer as a control was added, and the cells were then stimulated with 5 µg of LPS (lipopolysaccharide, obtained from Sigma).

In the same way as above, cells adjusted to 1×10⁶ cells per 48-well culture plate were stimulated with 0.5 µg of ConA (concanavalin A) in the presence or absence of γ-glutamylcysteine or glutathione. Thereafter, the cells were cultured in a CO₂ incubator at 37°C for 5 days, and the amounts of the antibodies in the supernatant were analyzed.

The amounts of IgA and IgG were measured by the ELISA method. The method for measuring IgG is as described below. 100 µl of a rat anti-mouse IgG antibody (Calbiochem, 1 µg/ml 50 mM sodium carbonate buffer, pH 8.5) solution was added to a 96-well ELISA plate, and the plate was incubated at 4°C overnight to coat each well with the anti-mouse IgG antibody. The plate was washed with PBST (phosphate buffered saline, 0.05% Tween20) three times and thereafter subjected to a blocking treatment with 200 µl of a PBS solution containing 2% BSA at 37°C for 1 hr.

The plate was washed with PBST three times, and 100 µl of the culture supernatant (diluted 9-fold with PBST containing 1% BSA) was added, followed by a reaction at 37°C for 2 hours. The plate was washed again with PBST four times. 100 µl of an alkaline phosphatase-conjugated anti-mouse IgG (rabbit) (BD Biosciences) solution diluted 2000-fold with PBST containing 1% BSA was added, followed by a reaction at 37°C for 1 hour. The plate was washed six times, and thereafter a color was developed with p-nitrophenyl phosphate. The reaction was stopped with 100 µl of 3 M NaOH / well, and the absorbance was measured at 405 nm.

IgA was measured in accordance with the same method as for IgG except that a rat anti-mouse IgA antibody (BD Biosciences) was used as the immobilized antibody, a biotinylated anti-mouse IgA antibody (rat) (BD Biosciences) was used as the labeling antibody, a 3,3',5,5'-tetramethylbenzidine solution (TMB substrate-developing solution, Sigma) was used as the chromogenic substrate, color development was stopped with 1 M sulfuric acid, and the absorbance was measured at 450 nm.
Statistical processing was performed by the Student's t-test, and a P value of 5% or less was considered significant.

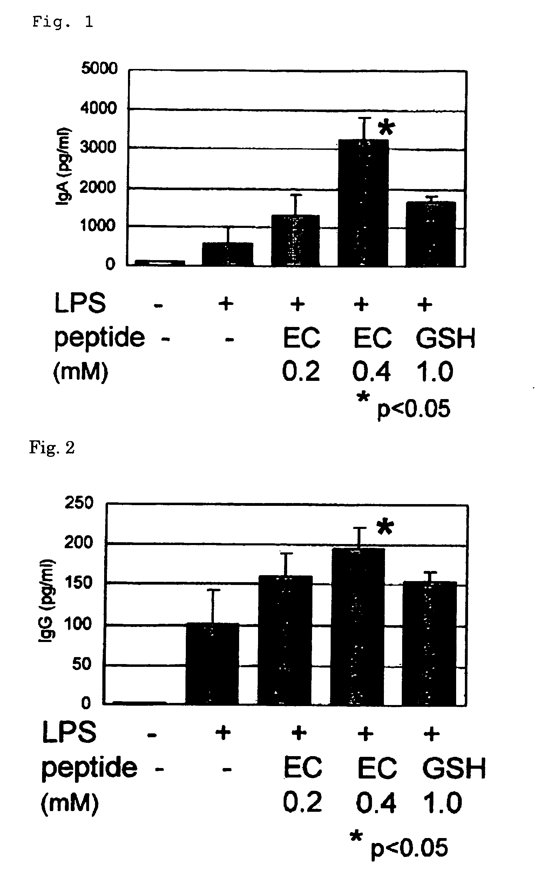

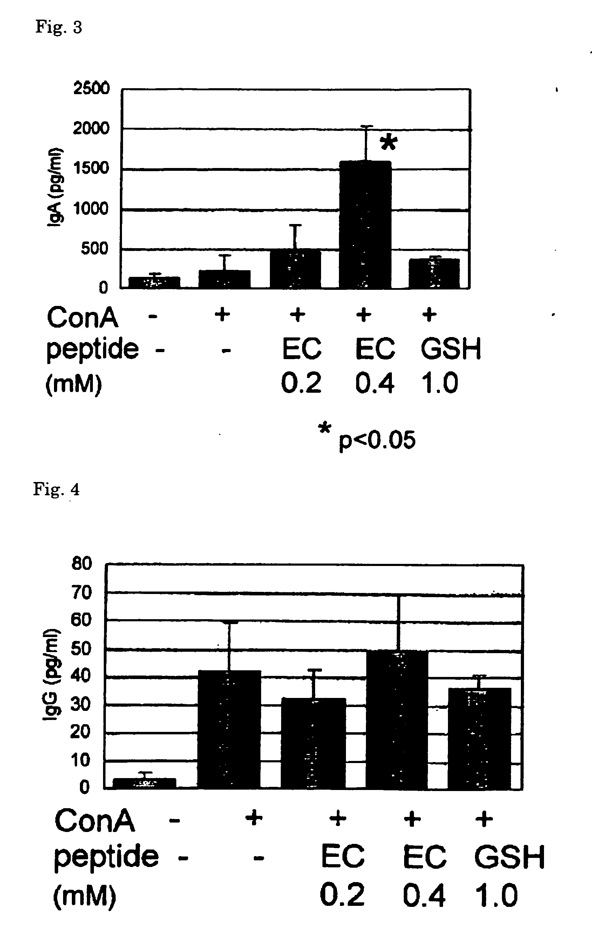

The results are shown in FIGS. 1 to 4. In the cases of both ConA stimulation and LPS stimulation, IgA production-promoting abilities of the peptides were significantly confirmed. On the other hand, the IgG production-promoting effect was lower than the IgA production-promoting effect, and it was suggested that γ-glutamylcysteine had a higher promoting effect for IgA production. In addition, it was shown that γ-glutamylcysteine had a particularly high promoting effect for IgA production as compared with glutathione.

### [Example 2]

Five-week-old BALB/c male mice (n=6) were fed for 1 week for habituation and then used in this experiment. During the feeding period, a commercially-available feed was used, and the mice were freely fed on the feed and water (tap water). γ-Glutamylcysteine was dissolved in sterilized physiological saline, and gavage administration was performed at 10 mg/kg B.W./day. The administration period was 7 days. During the administration period, the body weights were measured every day immediately before the administration to determine the dosage. There was no difference between the body weights of the different groups.

On day 7 after the start of the test, a necropsy examination was performed. Anesthesia was performed by interperitoneal administration with a pentobarbital formulation. At laparotomy, blood was collected from the abdominal aorta. The small intestinal contents were also collected.
Serum was collected from the collected blood, and the total IgA and total IgG concentrations were measured using an ELISA kit. The intestinal contents were diluted 10-fold with PBS and suspended sufficiently, and centrifugation was then performed to separate a supernatant. The total IgA concentration was measured by ELISA in the same way as in Example 1.

The results are shown in Table 1. In the table, γ-EC represents γ-glutamylcysteine. The table shows relative indices of the respective test groups with respect to the value of the control group to which γ-glutamylcysteine was not administered, which value was defined as 1. The amount of IgA present in the small intestine was found to increase by ingestion of the peptide. On the other hand, the amounts of serum IgA and serum IgG were found not to increase, and it was confirmed that the peptide had an effect of stimulating the immunoactivity in the intestinal mucosa.

**Table 1 Antibody-producing activity of peptide**

| Item | Non-administered group | γ-EC administered group |
|---|---|---|
| Small intestine IgA | 1.0 | 3.0 |
| Serum IgA | 1.0 | 0.3 |
| Serum IgG | 1.0 | 0.6 |

Relative indices with respect to the value of the non-administered control group defined as 1

### Industrial Applicability

The intestinal immune system stimulator of the present invention can stimulate the intestinal immune system safely and effectively. Further, the food or beverage of the present invention has an action of stimulating the intestinal immune system.

## Claims

1. An intestinal immune system stimulator comprising γ-glutamylcysteine.

2. The intestinal immune system stimulator according to claim 1, which is used as a medicament for the treatment or prevention of infection, diarrhea, polyp, tumor, enteritis, or allergy.

3. A food or beverage comprising γ-glutamylcysteine.

4. The food or beverage according to claim 3, comprising γ-glutamylcysteine in an amount of 0.000001% or more by mass.

5. The food or beverage according to claim 3 or 4, which has an effect of stimulating the intestinal immune system.
